# EUROPEAN PATENT APPLICATION

(11) **EP 3 306 559 A1**
(43) Date of publication of application: **11.04.2018**
(21) Application number: 16806933.4
(22) Date of filing: 07.06.2016
(51) Int. Cl.: G06Q 50/22, G06F 17/00, G06F 19/00

(54) **METHOD FOR THE UNIFIED REGISTRATION AND IDENTIFICATION OF BLOOD DONORS**

(30) Priority: 08.06.2015 ES 201530667 U; 06.11.2015 ES 201531611
(71) Applicant: Conectate Soluciones y Aplicaciones, S.L.U., 42002 Soria (ES)
(72) Inventor: SALA CANO, Nuria, 42002 Soria (ES); LATORRE LOPEZ, Fernando, 42002 Soria (ES)
(74) Representative: Danubia Patent & Law Office LLC
(86) International application number: PCT/ES2016/070428
(87) International publication number: WO 2016/198713

(57) **Abstract**

The invention relates to a method for the unified registration and identification of blood donors, comprising: generation of a donor profile associated with a non-transferable identification code, filing of the profile in a portable electronic device and storage of same in a database that can be accessed by different centres; identification of the donor by the centre with the code in order to access the profile, generation of an alarm in the event of an impediment, and automatic updating of the profile in the database; optionally, acquisition of analysis results, storage of same in the database and user notification, acquisition of relevant information from other medical centres and incorporation of said information into the profile, detection of the presence of users at donation points and notification of same, verification of the accuracy of the profile data and updating of same.

## Description

### OBJECT OF THE INVENTION

The invention, as expressed in the title of the present specification, refers to a unified registration procedure and
identification of blood donors, which has advantages and characteristics, which will be described in detail below, which involve an improvement of the current state of the technique.

The object of the present invention rests, in particular, in a
procedure whose purpose is to achieve the unification, for example, in all the centers of a certain territory, of the registration and identification of blood donors, among which it is not excluded to also include donors of plasma, platelets or red blood cells, which exist therein, in order to facilitate the exchange of information regarding
said donors and avoid the atomization and disintegration of data saving resources, said procedure consisting in generating, by the user his/herself through a computer application with a portable electronic device (mobile,
tablet, ...), or by the centers through the computer system, a profile with data from the donor associated with a code of
non-transferable identification, which is deposited and stored as a digital record in the database of a central computer system accessible by the different centers of said territory.

### FIELD OF APPLICATION OF THE INVENTION

The field of application of the present invention is the part of the health sector, particularly in the area of blood donation collection centers, encompassing at the same time, data management systems.

### BACKGROUND OF THE INVENTION

As is well known, the information regarding blood donors can be very extensive and varied; from the particularities of each
donor, relative to the blood group, age, donations made, etc. to the possible incidents or circumstances that can cause modification of their possibilities of remaining a temporary or definitive donor, for travel, illnesses, etc. as well as other conditions.

On the other hand, until now there has been no common and centralized system of obtaining such information from the centers and hospitals that collect blood from donors, which, although some of them provide a donor card that includes
very brief information, each center must collect and verify the information of the new donors.

The object of the present invention is therefore to avoid said drawback by developing a personal and non-transferable registration code and identification of the donors that can be utilized in a quick and practical manner by all of the centers of a territory, whether the donor has ever visited that center or another.

On the other hand, and as a reference to the current state of the art, it should be well-noted that, although different types of
identification systems, at least on behalf of the applicant, the existence of any that presents technical and constitutive characteristics similar to those presented by the invention claimed here is unknown.

### EXPLANATION OF THE INVENTION

The procedure of unified registration and identification of donors that the invention proposes, is configured, therefore, as a novelty within its
field of application, since according to its implementation the objectives outlined above are satisfactorily achieved, their characterizing details conveniently collected in the final claims accompanying the present specification.

Specifically, what the invention proposes, as indicated above, is a procedure for the registration and identification of blood donors developed with the aim of achieving the unification, in all the centers of a territory, of the registration system and identification of its donors and facilitate the exchange of information related thereto.

For this, said procedure essentially comprises the following:
- Generation of a donor profile associated with a non-transferable identification code, deposited in a portable electronic device (mobile, tablet, ...) and stored in a database accessible by the different centers.
- Identification of the donor by the system executed in the center.
   through the identification code for access to the profile of the donor, generation of an alarm if there is an impediment and automatic updating of the profile in the database by the different centers.

Optionally, it also contemplates:
- Capture of the results of the analysis, storing them in the database and notifying the user when the results are available.
- Capture of relevant information from other associated medical centers and incorporation of it into the donor profile.
- Detection of the presence of users near a donation point and notifying them.
- Verification of the accuracy of donor profile data and updating it.

Providing more detail, it should be noted that the code generated according to the procedure of the invention, which is unique and non-transferable for each donor, optionally consists of a QR code generated through a computer application through an electronic device portable (mobile, tablet, ...), and includes at least a series of characters or that which identifies and distinguishes it as unique, and information of the profile of the donor (type of blood, last donation, incompatibilities, ...), and that, once
generated, it is deposited and stored as a digital record in a database of a central computing medium accessible by all the different centers assigned thereto.

It should be noted that the code with the profile of the donor is generated either by the
donor his/herself through his/her electronic device with the software of the cited application or one of the donation centers, also through the application installed in any electronic device or computer.

With this, through the generation of said code, the identification of the donor by the computer equipment or electronic device, conveniently installed for that purpose in each center, can be made to connect to the central computer system and through the identification characters of the identification code to gain access to the details of the profile of each donor.

In case of existence of any impediment to blood being donated (the necessary time between donations has not passed, detected illness, ...), the central information system detects it via the information contained in the code, being able
generate an alarm (visual, sound) that alerts the corresponding personnel of this circumstance.

The generated code, in addition, allows one to vary the content of the information that it incorporates and, consequently, the automatic update of the profile
of the donor in the database of data accessible by the different centers (date of donation, amount donated, place of donation).

Optionally, the central computer system can capture and associate the code of each user with the result of laboratory analysis
responsible for the creation of the same after each donation and store them in the database accessible by the different centers. The code may incorporate all or part of said additional information that will be inserted into the donor's profile automatically by the central computer system in an automatic manner or a method that is selective to
the application.

In addition, the computer application may send a notification to the user's electronic device when the results are searchable.

Optionally, the computer system may capture relevant information for donations from other associated medical centers, for example in case of detection of a disease in a routine blood analysis, and incorporate said information into the profile of the donor.

Also, optionally, the generated code allows the system to automatically detect the presence of a nearby user (within a range of action) at a point or active donation center at said moment, allowing the generating of a notification to the user indicating, via
his/her mobile electronic device, the possibility of donating blood in the center near him or her.

If the donor profile that incorporates the code has been generated by the donor itself, optionally, the system can verify that the
data entered is authentic after capturing the result of the analysis of the laboratory in charge of the creation thereof and updating the data of the profile detail of the repeated donor as verified data.

The described unified registration code and identification of donors
consists, then, of an object of characteristics unknown until now for the purpose for which it is intended, reasons which, together with its practical utility, provide it with sufficient grounds to obtain the privilege of exclusivity that is requested.

### DESCRIPTION OF THE DRAWINGS

To complement the description that is being made and in order to
provide a better understanding of the characteristics of the invention, the present description is accompanied, as an integral part of the same, by a plan in which the following has been represented with an illustrative and nonlimiting character:
Figure number 1.- Shows, through a block diagram, the scheme of flow of operation of the code, according to the invention.

### PREFERRED EMBODIMENT OF THE INVENTION

In view of the described figures, and in accordance with the numeration adopted therein, it can be seen how the process comprises, essentially, the following phases and steps:
In a first phase (A):
   i) Generation by the user or a center, of a donor profile (type of blood, last donation, incompatibilities, ...) associated with a non-transferable identification code;
   ii) Deposit from said profile into a portable electronic device (mobile, tablet,...);
   iii) Storage of the profile in a database accessible by the different centers.
In a second phase (B):
   iv) Identification of the donor by the system executed in the center, through the non-transferable identification code for access to the details of the profile of the donor;
   v) Generation of an alarm (visual, sound) in case of existence of any impediment (the necessary time between donations has not passed, detected illness, ...);
   vi) Automatic update of the donor profile in the database accessible by the different centers (date of donation, amount donated, place of donation).

In addition, optionally, the procedure contemplates, in the use phase:
vii) Capture of the result of the analyses of the laboratory in charge of generating them and storage of them in the database accessible by the different centers.
viii) Sending of a notification to the user's electronic device when the results are available.

Optionally, the procedure also contemplates:
ix) Capture of relevant information from other associated medical centers (detection of a disease in a routine blood test).
x) Incorporation of this information into the donor profile.
xi) Automatic detection of the presence of a nearby user (within a range of action) at an active donation point at that time.
xii) Generation of a notification to the user indicating the possibility of donating blood in the center near to him/her.

Finally, if the donor profile has been generated by the user, optionally the procedure contemplates:
xiii) Data entered is authentic after capturing the result of the analysis of the laboratory in charge of the creation and updating the data of the profile detail of the repeated donor as verified data.
xiv) Updating the data of the profile detail as
   verified data.

Having sufficiently described the nature of the present invention, as well as the mode of putting it into practice, it is not considered necessary to make the explanation more extensive so that any expert in the field
may understand its scope and the advantages that derive therefrom, stating that, within its essentiality, it may be carried out, in practice, in other modes of realization that differ in detail from that indicated by way of example, and to which it may equally reach the protection that is sought, provided that it is not altered, changed or modified in its fundamental principal.

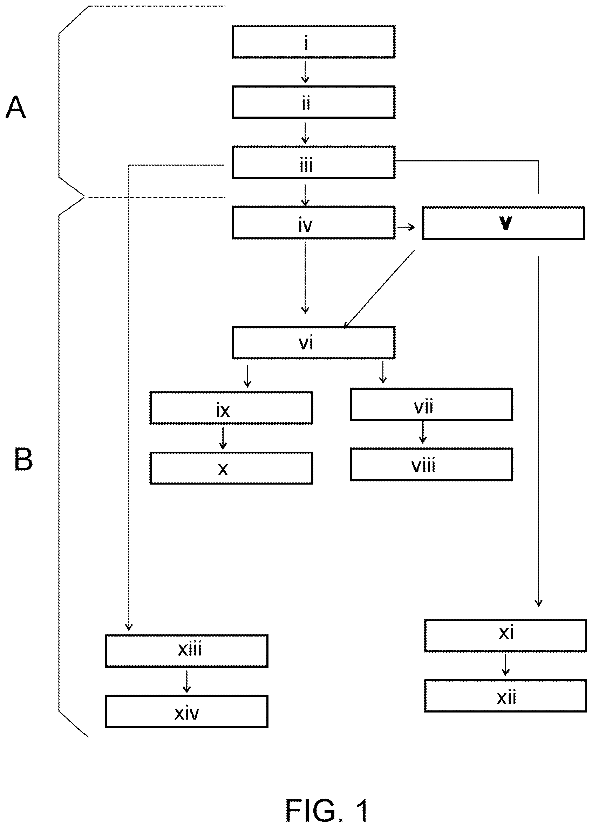

## Claims

1. Procedure of unified registration and identification of blood donors, **characterized in that** it comprises:
In a first phase (A):
i) Generation by the user or a center, of a donor profile (type of blood, last donation, incompatibilities, ...) associated with a non-transferable QR identification code;
ii) Deposit from said profile into a portable electronic device (mobile, tablet...);
iii) and storage thereof in a database accessible by the different centers.
In a second phase (B):
iv) Identification of the donor by the system executed in the center through the non-transferable QR identification code for access to the details of the donor profile;
v) Automatic generation of an alarm (visual, sound) in case of existence of any impediment (the necessary time between donations has not passed, detected illness, ...);
vi) Automatic update by the donor profile system in the database accessible by the different centers (date of the
donation, amount donated, place of donation).

2. Procedure of unified registration and identification of blood donors, according to claim 1, **characterized in that** it also includes:
vii) Automatic capture by the system of the results of the analyses by the laboratories in charge of the creation thereof, and storage thereof the database, accessible by the different centers.
viii) Sending of a notification to the user's portable electronic device when the results are available.

3. Procedure of unified registration and identification of blood donors, according to claim 1 6 2, **characterized in that** it also comprises:
ix) Capture of relevant information from other associated medical centers (detection of a disease in a routine blood test).
x) Automatic incorporation of this information into the donor profile by the system.

4. Procedure for unified registration and identification of donors of blood according to any one of claims 1 to 3, **characterized in that** it also includes:
xi) Automatic detection by the system of the presence of a nearby user (within a range of action) at a
donation point at that time.
xii) Generation of a notification to the user indicating the possibility of donating blood in the center next to him.

5. Procedure of unified registration and identification of blood donors, according to any of claims 1 to 4, **characterized in that**, when the donor profile has been generated by the user, it also includes:
xiii) Automatic verification by the system that the entered data is authentic after capturing the result of the analysis of the laboratory in charge of the creation thereof;
xiv) Automatic incorporation of this information into the donor profile by the system
of donor profile as verified data.
